# EUROPEAN PATENT SPECIFICATION

(11) **EP 3 165 233 B1**
(45) Date of publication and mention of the grant of the patent: **18.08.2021**
(21) Application number: 15183024.7
(22) Date of filing: 28.08.2015
(51) Int. Cl.: A61K 38/16, A61L 26/00, A61K 47/26, A61K 9/06, A61K 36/062, A61P 17/02

(54) **BIOMATERIAL FOR TREATMENT OF ACUTE AND CHRONIC SKIN WOUNDS**
BIOMATERIAL ZUR BEHANDLUNG VON AKUTEN UND CHRONISCHEN HAUTWUNDEN
BIOMATÉRIAU POUR LE TRAITEMENT DE LÉSIONS CUTANÉES AIGUËS ET CHRONIQUES

(43) Date of publication of application: 10.05.2017
(73) Proprietor: Latvijas Universitate, 1586 Riga (LV)
(72) Inventor: Ramata-Stunda, Anna, LV-1011 Riga (LV); Boroduskis, Martins, LV-5232 "Slani" (LV); Makarenkova, Galina, LV-1001 Riga (LV); Petrina, Zaiga, LV-1007 Riga (LV); Nikolajeva, Vizma, LV-1021 Riga (LV); Telle, Vika, LV-4401 Gulbene (LV); Muiznieks, Indrikis, LV-1024 Riga (LV); Jansone, Baiba, LV-1028 Riga (LV)
(74) Representative: Fortuna, Aleksandra

(56) References cited:
- EP-A1- 2 581 079
- WO-A1-01/06973
- WO-A1-2005/067944
- WO-A1-2015/015407
- WO-A2-02/39948
- CN-A- 1 927 414
- CN-A- 101 313 915
- JP-A- 2001 017 532
- JP-A- 2001 212 222
- LV-B- 13 959
- US-A1- 2010 316 683
- None

## Description

### Technical field

This invention relates to medical devices and compositions for skin regeneration and healing of acute and chronic wounds.

### Prior art

Fungal and plant polysaccharides and glycoproteins are the most known natural immunomodulators that have been used as topical agents in wound healing. (Du et al. 2013).

There is current experience of use of natural polysaccharide and glycoprotein immunomodulators in wound healing as a single agents or in combination of biomaterials.

Patent US7022668 B2 describes the use of glycoprotein produced by *Pseudoalteromonas antartica* for topical or mucous application in the treatment and re-epithelialization of wounds.

Beta-glucan, one of the best characterized fungal immunomodulators, has been incorporated in collagen matrix and used as a temporary coverage for adult and pediatric partial thickness burns with reported good results. Observed advantages of this type of coverage include reduction of pain, improved healing, and better scar appearance (Delatte et al. 2001).

Beta-glucan such as zymosan has been shown to be beneficial in wound healing as it promotes collagen synthesis (Wei et al. 2002). Ability of beta-glucans to promote wound healing has also been shown in diabetic experimental animals (Berdal et al. 2007).

Use of fungal glycoprotein adenylate deaminase (AMPD) containing hydrogel for treatment of skin ulcers has been described in the patent LV13959. AMPD used for production of hydrogel in this patent was isolated from laboratory strain of soil fungus

### Penicillium lanoso-viride 8D.

Previous studies have shown that glycoprotein AMPD isolated from *P.lanoso-viride* 8D has immunomodulatory characteristics - it modulates both cellular and humoral immunity. AMPD promotes proliferation of lymphocytes, activates murine peritoneal macrophages (Nikolajeva et al., 1999), stimulates natural killers and possesses antitumor activity (Zak et al., 1986), enhances resistance to infections (Nikolajeva et al., 1996), inhibits experimental autoimmune encephalomyelitis (Nikolajeva et al., 2000).

Current experience of glycoprotein use as topical skin healing agents together with known biological activities of *P.lanoso-viride* 8D glycoprotein AMPD have been preconditions for us to optimize glycoprotein isolation method and use of it to functionalize hyaluronic acid based biomaterial for wound healing.

Hyaluronic acid (HA) is highly hydrophilic glycosaminoglycan, which is an essential component of the animal tissue extracellular matrix. Previous experience shows usefulness of HA hydrogels and scaffolds in combination with other materials and/ or active substances in wound closure, re-epithelialization and neovascularization (Wang et al. 2013., Yan et al. 2013., Cerqueira et al. 2014.).

Current experience of preparation of crosslinked HA gels is described in several patents, for example, United States patent No. 4716224, patent No. US005783691A and patent No. EP 2236523A1.

There is current experience of use of HA in wound healing. Patent No. US6541460B2 describes method for use of hyaluronic acid in open wound management by topical application by syringe through a thin film dressing. Method for healing burns and wounds in mammals with biomaterial containing the combination of HA and a serine protease inhibitor is described in patent EP1270013A1.

### Disclosure of the invention

The present invention relates primarily to production of functionalized biodegradable, hyaluronic acid based biomaterial, aimed at skin wound healing.

A biomaterial for treatment of acute and chronic skin wounds is proposed being a hyaluronic acid based functionalized biomaterial comprising crosslinked hyaluronic acid and hyaluronic acid gel. The v/v ratio of crosslinked hyaluronic acid and hyaluronic is 1:2. The molecular mass of hyaluronic acid used for production of both crosslinked and gel component of the biomaterial is within the range of 2,00 - 2,20 MDa. According to the invention, the crosslinked component of the biomaterial is produced using 1,4-butanediol diglycidil ether as a crosslinking agent. The biomaterial is functionalized using purified glycoprotein fraction, which is isolated form filamentous fungus *Penicillium lanoso-viride.* The dominating N-glycans of the purified glycoprotein fraction are Man7GlcNAc, Man8GlcNAc, Man9GlcNAc. The preferred concentration of the purified glycoprotein in the biomaterial is 15-25 µg/ml, more preferably 20 µg/ml. The proposed biomaterial can be used in treatment of acute and chronic (e.g. diabetic or venous) wounds, by direct application of the biomaterial on cleaned wound. The proposed biomaterial can be used in medical devices or kits for treating skin wounds.

### Brief description of drawings

Fig. 1 - diagram of migration of HaCaT keratinocytes, expressed as % of initial scratch wound area. Fig. 1A shows the effect of purified glycoprotein after direct addition to the keratinocytes in scratch test; Fig. B - immune mediated response - glycoprotein is not added directly, but perhipheral blood mononuclear (PBMNC) cell incubation medium after stimulation with glycoprotein is added to the keratinocytes in the scratch test.
Fig. 2 - diagram showing effect of hyaluronic acid based biomaterial (combination of crosslinked and gel forms in ration 1:2), functionalized with 20 µg/ml glycoprotein (designated in the diagram as HA + Glycoprot 20) on proliferation of HUVEC cells over the period of 126h; functionalized biomaterial shows stimulatory effect on proliferation of endothelial cells.
Fig. 3 - diagram showing changes in wound area (cm²) of experimental wounds in Wistar male rats over 12 day period.
Fig. 4 - pictures showing wound area at the beginning of *in vivo* wound healing experiment in Wistar male rats and on day 12 of the experiment.

### Purification of immunomodulatory glycoproteins

For isolation of glycoprotein rich fraction *Penicillium lanoso-viride* 8D was cultivated on the surface of malt extract broth (30g/L malt extract) at room temperature. As production of previously characterized AMPD is most pronounced in *P.lanoso-viride* 8D at sporulation stage, mycelia for extraction of glycoproteins were collected by filtration on 5^{th} day of cultivation.

Mycelium was ground in sterile 0.05M potassium phosphate buffer solution (pH 6.0) in bead mills (speed 700 rpm, for total of 15 minutes with intermittent cooling) and homogenate was centrifuged for 40 min at 4 °C at speed 4000 x g. Supernatant was collected for glycoprotein precipitation. Supernatant was fractioned by stepwise ammonium sulphate precipitation:
1) 33 g of ammonium sulphate was slowly added per 100 ml of extract (50% saturation of ammonium sulphate at 4 °C) followed by centrifugation for 40 minutes at 4 °C at speed 4000 x g; pellet was discarded and supernatant used for further precipitation;
2) 22 g of ammonium sulphate is added to supernatant (77% saturation of ammonium sulphate at 4 °C) followed by centrifugation for 40 minutes at 4 °C at speed 4000 x g; supernatant was discarded and pellet resuspended in PBS

Dialysis of suspension against 200 volumes of 0.05M potassium phosphate buffer solution was performed overnight. Protein concentration in samples was determined spectrophotometrically (A₂₈₀ₙₘ). Acquired concentrations were in range 20 - 40 mg/ml. Afterwards glycoprotein fraction was purified by gel filtration on Sephadex G-200 column (dimensions 2.5 x 90 cm). Fractions are collected from the column with 0.05M potassium phosphate buffer solution at flow rate 0.25 - 0.3 ml/min.

### Biochemical characterization of purified P.lanoso-viride glycoprotein fractions

Isolated fractions are characterized by presence of high molecular weight glycoproteins and enzymatic activity of adenosine monophosphate deaminase (AMPD).

AMPD activity, characteristic to purified glycoprotein fraction, was determined in each collected fraction - one unit of enzymatic activity was determined as quantity of AMPD that deaminated 1 µmol of 5'-AMP to 5'-IMP per minute at 37 °C (pH 6.0). (Nikolajeva et al., 1996). Protein concentrations of the fractions were determined spectrophotometrically. Isolation yields 0.5-1.5 mg/ml protein. For purified fractions the AMPD enzymatic activity is within the range of 20-60 u/mg protein.

N-glycosylation profile was characterizes for isolated glycoproteins. High molecular weight glycoprotein bands were excised form polyacrylamide gel slices, reduced, alkylated and deglycosylated with PNgase F (100u /500µl, buffer pH8) overnight at 37°C. The N-glycans were extracted with water and 50% acetonitrile. Glycans were desalted and dissolved in 30µl of water and applied to the column. Salts were then washed off with water and the glycans eluted in 50µl of 80% acetonitrile in 0.1%TFA before being dried in vacuum. Glycan samples were permethylated in mix DMSO/ NaOH/ ICH3 for 20min. One volume of water was added and the N-glycans were extracted in two volumes of chloroform. The chloroform was washed 5 times in water then dried. One µl of N-glycans dissolved in 50% methanol, 5mM sodium acetate was mixed with 1 µl of DHB matrix (10 mg/ml in Methanol/H2O), spotted on an analysis plate and dried. MALDI-TOF spectra were acquired in a reflector positive mode. N-glycan structures present in purified glycoprotein fractions are oligomannose type glycans Man5 GlcNAc2 to Man10 GlcNAc2, with Man7GlcNAc, Man8GlcNAc, Man9GlcNAc being the dominating glycans.

### Production of hyaluronic acid based biomaterial and it's combination with purified glycoprotein fraction

Production of spread type polymer hydrogel combines use of hyaluronic acid (HA) and cross-linked hyaluronic acid without complex purifying steps. Cross-linking agent with free-state functional group in product is < 1 ppm. The method contains three steps: 1) production of cross-linked HA as described by Schante et al. 2011 and Kenne et al. 2013, using pharmaceutical grade sodium hyaluronate (molecular weight 2,00 - 2,20 MDa, bacterial endotoxins < 0.050 IU/mg) concentration of 20 w/v %, base concentration of 0.25 N, low reaction temperature of 30°C and cross-linking agent (1,4-butanediol diglycidil ether) concentration of 1 v/v%. In brief: the suspension was placed in thermostat at 30°C for 5 days reaction time. The cross-linked HA under laminar flow was cut into smaller pieces, then PBS of pH 7, 4 to get suspension of 18mg/ml was added. Suspension was homogenized using filtration through stainless steel sieve of pore size 103 µm. pH was regulated and adjusted to suitable physiology (pH 7,4) using 6N hydrogen chloride solution or 5N sodium hydroxide solution. HA suspension was sterilized by autoclaving. 2) The second step is production of 1,5% or 15mg/ml HA gel from the same MW, source and type of sodium hyaluronate. Suspension was obtained under constant stirring by magnetic stirring bar for 30 min at 80°C, pH was regulated and adjusted to pH 7,4, then sterilized by autoclaving. 3) The third step is to mix the cross-linked HA 18mg/ml at a ratio of 1:2 with HA gel 15 mg/ml and adding of 20 µg/ml purified glycoprotein fraction in aseptic conditions. Then hyaluronic acid based biomaterial is filled in sterile syringes and ready to use for applications to skin wounds.

### In vitro effect on skin keratinocytes and endothelial cells

To evaluate the effects of both the immunomodulatory glycoprotein fraction alone and a hyaluronic acid based biomaterial containing it HaCaT keratinocytes and human umbilical vein endothelial cells (HUVEC) where cultivated in the presence of glycoprotein and/or biomaterial containing it. Additionally indirect, immune-mediated, effect of the glycoprotein and biomaterial on dermal and epidermal cells was assessed by testing first stimulating peripheral blood mononuclear cells (MNC) with glycoprotein and afterwards adding glycoprotein conditioned MNC incubation medium to keratinocyte cultures. Effects on cell proliferation and migration were assayed using IncuCyte Live-Cell Imaging System (Essen Bioscience) and IncuCyte Zoom software. In short, equal numbers of the cells were seeded in 24 of 96 well plates and the kinetics of cell growth was monitored using the IncuCyte integrated confluence algorithm, where confluence is a surrogate for cell number. Alternatively cell migration was assayed in scratch tests. In short, the rate of cell migration into a scratch wound in a confluent monolayer of cells was determined from average wound widths measured at 1 hour intervals using the IncuCyte system (Essen Bioscience). Wounds were made in cells cultured in 96-well cell cultivation plates (Essen Bioscience) using the WoundMaker device (Essen Instruments).

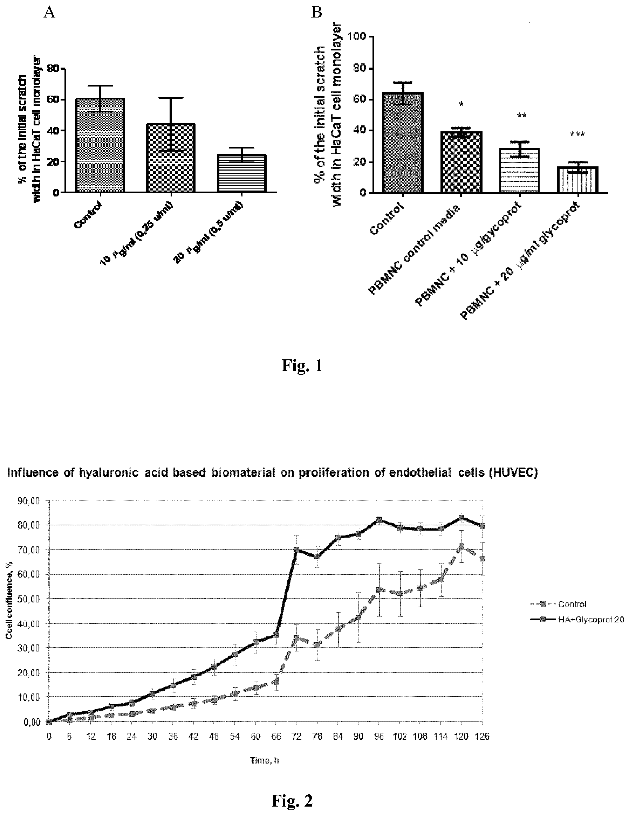

Changes in scratch wound width is was assessed 48h after direct addition of glycoprotein (Fig. 1A), and 16 hours after addition of PBMNC conditioned media (Fig. 1B). Significant reduction of wound area is seen in both cases - after direct addition of glycoprotein and after addition of glycoprotein stimulated PBMNC cultivation media.

Experimental data show that purified glycoprotein fractions have positive effect on keratinocyte migration and proliferation as scratch wound area after addition of glycoprotein is closed faster, compared to control. In addition, the effect is even more pronounced in case when glycoprotein stimulated MNC cultivation media is added to the keratinocyte cultures. Both tests show that effect is dose dependent (see Fig.1). Ability to stimulate both directly and indirectly epidermal cell migration approves the ability of the glycoprotein to promote wound healing. Moreover the indirect, immune-mediated, stimulatory activity indicates on the high potential of the glycoprotein to promote healing of chronic wounds, where apart from endogenous skin cell stimulation it is important to regulate immune response. The immunoregulating acitivities of the glycoprotein have been described already before (Nikolajeva *et al,* 1996, Nikolajeva *et al,* 1999).

In endothelial cell cultures (HUVECs) combination of glycoprotein fraction with hyaluronic acid (mixture of cross-linked and gel at the ration of 1:2) promotes cell proliferation (see Fig. 2). These experimental results further support the applicability of the combination of glycoprotein and hyaluronic acid in treatment of both chronic and acute wounds. The ability to stimulate growth of endothelial cells is of great importance in treatment of chronic wounds where angiogenesis is suppressed.

### Effect on wound healing in vivo

To evaluate *in vivo* wound healing efficacy of the new biomaterial (1:2 mix of crosslinked and gel hyaluronic acid with added glycoprotein fraction at concentration 20 µg/ml) experimental skin wound models where created using Wistar male rats. In short, 75 mg/kg 10% ketamine, 15 mg/kg 2% xilazine anesthesia was administered prior to creation of skin wounds. Two wounds in each animal's dorsal area where created using punch biopsy devices (d=8mm). To avoid wound contraction silicone splints (d=10 mm) where fixed to the wound edges by sewing on with surgical monofilament sutures (size 6/0). Hyaluronic acid (mix of crosslinked and gel at ratio 1:2), and hyaluronic acid combined with 20 µg/ml glycoprotein was applied to skin wounds every second day. Sterile phosphate buffered saline (pH 7,4) was applied in control group. After application of the test materials wounds were covered with contact dressing (Mepitel One Safetac) and secondary dressing to protect the wound. Each experimental group consisted of 10 animals. Photographs of wounds to assess the dynamics of the healing where taken every second day and area of the wound measured.

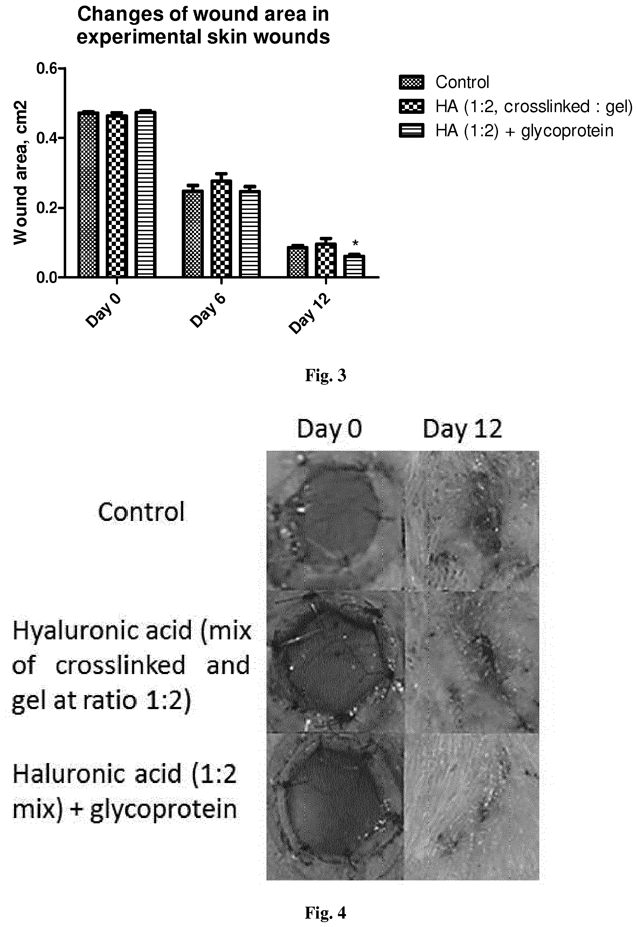

Results (see Fig.3 and Fig.4) show that biomaterial where crosslinked and gel hyaluronic acid is combined with purified glycoprotein promote wound healing *in vivo.* Functionalized biomaterial (designated as HA (1:2)+glycorprotein) reduces wound area and promotes reepitelization rate statistically significantly compared both to control and to biomaterial without added glycoprotein (Fig. 3).

The results indicate the applicability of the new biomaterial in clinical practice to promote healing of skin wounds.

### REFERENCES

1. Berdal M., Appelbom H.I., Eikrem J.H., Lund A., Zykova S., Busund L.T., Seljelid R., Jenssen T. 2007. Aminated beta-1,3-D-glucan improves wound healing in diabetic db/db mice. Wound Repair Regeneration, 15: 825 - 32.
2. Cerqueira M.T., da Silva L.P., Santos T.C., Pirraco R.P., Correlo V.M., Marques A. P.M., Reis R.L. 2014. Human skin cell fractions fail to self-organize within a gellan gum/hyaluronic acid matrix but positively influence early wound healing. Tissue Engineering, 20 (9; 10): 1369 - 1378.
3. Delatte S.J., Evans J., Hebra A., Adamson W., Othersen H.B., Tagge E.P. 2001. Effectiveness of beta-glucan collagen for treatment of partial-thickness burns in children. Journal of Pediatric Surgery, Jan 36(1): 113 - 8.
4. Du B, Bian Z, Xu B. 2013. Skin Health Promotion Effects of Natural Beta-Glucan Derived from Cereals and Microorganisms: A Review. Phytother. Res. (2013) doi: 10.1002/ptr.4963
5. Kenne L., Gohil S., Nilsson E.M, Karlsson A., Ericsson D., Kenne A.H., Nord L.I. 2013. Modification and cross-linking parameters in hyaluronic acid hydrogels- Definitions and analytical methods. Carbohydrate Polymers, 91 (1): 410 - 418.
6. Nikolajeva V., Eze D., Kamradze A., Indulena M., Muiznieks I. 1996. Protective effect of adenylate deaminase (from Penicillium lanoso-viride) against acute infections in mice. Immunopharmacology, 35: 163 - 169.
7. Nikolajeva V., Eze D., Petrina Z., Muiznieks I. 1999. Activation of mice peritoneal macrophages by adenylate deaminase from Penicillium lanoso-viride. Proceedings of the Latvian Academy of Sciences, sect. B, 53 (1): 12 - 15.
8. Nikolajeva V., Eze D., Petrina Z., Muiznieks I. 2000. Treatment of experimental autoimmune encephalomyelitis with adenylate deaminase from Penicillium lanoso-viride. Journal of Autoimmunity, 14: 107 - 113.
9. Schanté C.E., Zuber G., Herlin C., Vandamme T.F. 2011. Chemical modifications of hyaluronic acid for the synthesis of derivatives for a broad range of biomedical applications. Carbohydrate Polymers, 85 (3): 469 - 489.
10. Wang H.M., Chou Y.T., Wen Z.H., Wang Z.R., Chen C.H., Ho M.L. 2013. Novel biodegradable porous scaffold applied to skin regeneration. PloS ONE, 8(6): e56330.
11. Wei D., Zhang L., Williams D.L., Browder I.W. 2002. Glucan stimulates human dermal fibroblast collagen biosynthesis through a nuclear factor-1 dependent mechanism. Wound Repair Regeneration, 10: 161 - 8.
12. Yan S., Zhang Q., Wang J., Liu Y., Lu S., Li M., Kaplan D.L. 2013. Silk fibroin/ chondroitin sulfate/hyaluronic acid ternary scaffolds for dermal tissue reconstruction. - Acta Biomaterialia, 9: 6771 - 6782.

### PATENT CITATIONS

1. Duena A.P., Garces J.G., Sanchez J.G,, Anton J.M.G., Marano C.M., Del Pozo M.R., Coma S.V. 2006. US7022668 B2, Use of a glycoprotein for the treatment and reepithelialisation of wounds.
2. Lezdey D., Lezdey J. 2003. EP1270013A1, Topical wound healing compositions comprising hyaluronic acid and a serine protease inhibitor.
3. Malson T., Lindquist B. 1998. Patent No. US005783691A, Crosslinked hyaluronate gels, their use and method for producing them.
4. Nikolajeva V., Artjuha M., Eze D., Petrina Z., Babarikins D., Muiznieks I. LV 13959B, Adanylate deaminase containing hydrogel and its use in treatment of skin ulcers.
5. Petito G.D. 2003. US6541460B2, Method for use of hyaluronic acid in wound management.
6. Sakurai K., Ueno Y., Okuyama T. 1987. Patent No. 4716224, Crosslinked hyaluronic acid and its use.
7. Tor-Chern Ch., Li-Siu Ch. 2010. EP2236523 A1, Method for producing cross-linked hyaluronic acid.

## Claims

1. A biomaterial for treatment of acute and chronic skin wounds being a hyaluronic acid based functionalized biomaterial comprising crosslinked hyaluronic acid and hyaluronic acid gel, wherein
- the crosslinked hyaluronic acid is mixed with hyaluronic acid gel in v/v ratio of 1:2;
- the molecular mass of hyaluronic acid used for production of both crosslinked and gel component of the biomaterial is within the range of 2,00 - 2,20 MDa;
- the crosslinked component of the biomaterial is produced using 1,4-butanediol diglycidil ether as a crosslinking agent;
- the biomaterial being functionalized using purified glycoprotein fraction, wherein the concentration of the purified glycoprotein in the biomaterial is 15-25 µg/ml;
- the glycoprotein component of the biomaterial is isolated form filamentous fungus *Penicillium lanoso-viride* by stepwise precipitation with ammonium sulphate followed by purification by gel filtration on Sephadex G-200 column;
- and wherein the dominating N-glycans of the purified glycoprotein fraction are Man7GlcNAc, Man8GlcNAc, Man9GlcNAc.

2. The biomaterial according to claim 1, wherein the purified glycoprotein fraction possesses adenosine monophosphate deaminase enzymatic acitivity.

3. A medical device for treating a skin wound comprising a biomaterial according to any claim from 1 to 2.

4. A kit for treating a skin wound comprising a biomaterial according to any claim from 1 to 2.

## Patentansprüche

1. Biomaterial zur Behandlung akuter und chronischer Hautwunden, bestehend aus einem funktionalisierten Biomaterial auf Hyaluronsäurebasis, vernetzter Hyaluronsäure und Hyaluronsäuregel, wobei
- die vernetzte Hyaluronsäure mit Hyaluronsäuregel im Verhältnis von 1:2 gemischt wird;
- die molekulare Masse der Hyaluronsäure, die für die Herstellung sowohl der vernetzten als auch der gelartigen Komponente des Biomaterials verwendet wird, im Bereich von 2,00 - 2,20 MDa liegt;
- die vernetzte Komponente des Biomaterials unter Verwendung von 1,4-Butandioldiglycidilether als Vernetzungsmittel hergestellt wird;
- das Biomaterial unter Verwendung einer gereinigten Glykoproteinfraktion funktionalisiert wird, wobei die Konzentration des gereinigten Glykoproteins im Biomaterial 15-25 µg/ml beträgt;
- die Glykoproteinkomponente des Biomaterials aus dem Fadenpilz *Penicillium lanoso-viride* durch schrittweise Fällung mit Ammoniumsulfat und anschließender Reinigung durch Gelfiltration auf einer Sephadex G-200-Säule isoliert wird;
- wobei es sich bei den dominierenden N-Glykanen der gereinigten Glykoproteinfraktion um Man7GlcNAc, Man8GlcNAc und Man9GlcNAc handelt.

2. Biomaterial nach Anspruch 1, wobei die gereinigte Glykoproteinfraktion eine enzymatische Aktivität von Adenosinmonophosphatdesaminase besitzt.

3. Medizinisches Gerät zur Behandlung einer Hautwunde, umfassend ein Biomaterial nach einem der Ansprüche 1 bis 2.

4. Kit zur Behandlung einer Hautwunde, umfassend ein Biomaterial nach einem der Ansprüche 1 bis 2.

## Revendications

1. Biomatériau pour le traitement de plaies cutanées aiguës et chroniques, étant un biomatériau fonctionnalisé à base d'acide hyaluronique comprenant de l'acide hyaluronique réticulé et un gel d'acide hyaluronique, dans lequel
- l'acide hyaluronique réticulé est mélangé à du gel d'acide hyaluronique dans rapport volume/volume de 1:2 ;
- la masse moléculaire de l'acide hyaluronique utilisé pour la production à la fois des composants réticulé et en gel du biomatériau est dans la plage de 2,00 à 2,20 MDa ;
- le composant réticulé du biomatériau est produit en utilisant de l'éther diglycidylique de 1,4-butanediol en tant qu'agent de réticulation ;
- le biomatériau étant fonctionnalisé en utilisant une fraction de glycoprotéine purifiée, dans lequel la concentration de la glycoprotéine purifiée dans le biomatériau va de 15 à 25 µg/ml ;
- le composant glycoprotéine du biomatériau est isolé d'un champignon filamenteux *Penicillium lanoso-viride* par précipitation par étapes avec du sulfate d'ammonium suivie par une purification par filtration sur gel sur une colonne Sephadex G-200 ;
- et dans lequel les N-glycanes dominants de la fraction de glycoprotéine purifiée sont Man7GlcNAc, Man8GlcNAc, Man9GlcNAc.

2. Biomatériau selon la revendication 1, dans lequel la fraction de glycoprotéine purifiée possède une activité enzymatique d'adénosine monophosphate désaminase.

3. Dispositif médical pour traiter une plaie cutanée comprenant un biomatériau selon l'une quelconque revendication de 1 à 2.

4. Trousse pour traiter une plaie cutanée comprenant un biomatériau selon l'une quelconque revendication de 1 à 2.
